(19) Europäisches Patentamt / European Patent Office / Office européen des brevets

(11) **EP 3 248 467 A1**

(12) **EUROPÄISCHE PATENTANMELDUNG**

(43) Veröffentlichungstag:
**29.11.2017   Patentblatt 2017/48**

(21) Anmeldenummer: **16171275.7**

(22) Anmeldetag: **25.05.2016**

(51) Int Cl.:
**A23D 7/005** (2006.01)     **A61K 31/202** (2006.01)
**C07C 51/00** (2006.01)     **C07C 57/03** (2006.01)
**C11C 1/02** (2006.01)     **A23L 33/17** (2016.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Evonik Technochemie GmbH**
**69221 Dossenheim (DE)**

(72) Erfinder:
• KNAUP, Günter
  **63486 Bruchköbel (DE)**
• LATINOVIC, Milan
  **63667 Nidda (DE)**
• LOTZ, Jörg
  **36148 Kalbach (DE)**
• SCHWARM, Michael
  **63755 Alzenau (DE)**

(54) **VERFAHREN ZUR HERSTELLUNG EINER ZUSAMMENSETZUNG ENTHALTEND OMEGA-3-FETTSÄURE-L-LYSIN-SALZE**

(57)     Die vorliegende Erfindung betrifft ein Verfahren zur Herstellung einer Zusammensetzung enthaltend ein oder mehrere Omega-3-Fettsäure-L-Lysin-Salz(e), die durch dieses Verfahren erhältliche oder erhaltenen Zusammensetzung, sowie die Verwendung dieser Zusammensetzung für die Herstellung von Nahrungsmitteln, Nahrungsergänzungsmitteln oder pharmazeutischen Produkten.

EP 3 248 467 A1

**Beschreibung**

Aufgrund einer umfangreichen, über die letzten Jahrzehnte zusammengetragenen Sammlung wissenschaftlicher Nachweise wurden zahlreiche gesundheitliche Vorteile mit der ergänzenden Einnahme von mehrfach ungesättigten Fettsäuren (PUFAs) in Verbindung gebracht. Die Prävention von Herz-Kreislauf-Erkrankungen und die Symptomreduzierung von Entzündungen gehören zu den wichtigsten Beispielen, wobei u.a. auch die Vorbeugung der Promotions- und der Progressionsphase einiger Krebsarten, die Senkung des Blutdrucks und des Cholesterinspiegels sowie positive Effekte in der Behandlung von Depression und Schizophrenie, Alzheimer, Dyslexie, Aufmerksamkeitsdefizitsyndrom und Hyperaktivität berichtet wurden. Da man davon ausgeht, dass einige PUFAs wesentlich für die Entwicklung des Gehirns, des Nervensystems und der Augen sind, wird außerdem heutzutage Säuglingsnahrung routinemäßig mit bestimmten PUFAs angereichert.

Die Herstellung von Nahrungsmitteln, Nahrungsergänzungsmitteln und pharmazeutischen Produkten mit PUFAs wird jedoch durch deren Oxidationsempfindlichkeit erschwert. Die Oxidation der PUFAs hat negative nahrungsphysiologische und organoleptische Folgen, wie Veränderungen des Nährstoffgehaltes durch Zerstörung wichtiger Fettsäuren; Ranzigkeit, die Fehlaromen und ausgeprägte Gerüche erzeugt; Farbveränderungen, wie etwa das Dunkelwerden von Fetten und Ölen; sowie Aromaverlust. Der oxidative Abbau von PUFAs erzeugt eine komplexe Mischung flüchtiger sekundärer Oxidationsprodukte, und diese erzeugen besonders unangenehme Fehlaromen.

Im Rahmen der vorliegenden Erfindung werden die Begriffe "PUFA" und "mehrfach ungesättigte Fettsäure" synonym benutzt und wie folgt definiert: Fettsäuren werden nach der Länge und der Sättigung ihrer Kohlenstoffkette eingeteilt. Kurzkettige Fettsäuren haben 2 bis etwa 6 Kohlenstoffatome und sind typischerweise gesättigt oder ungesättigt. Mittelkettige Fettsäuren haben etwa ab 6 bis etwa 14 Kohlenstoffatome und sind ebenfalls typischerweise gesättigt oder ungesättigt. Langkettige Fettsäuren haben etwa ab 16 bis 24 oder mehr Kohlenstoffatome und können gesättigt oder ungesättigt sein. In längerkettigen Fettsäuren kann es eine oder mehrere ungesättigte Stellen geben, was zu den Begriffen "einfach ungesättigt" bzw. "mehrfach ungesättigt" führt. Im Kontext der vorliegenden Erfindung werden langkettige mehrfach ungesättigte Fettsäuren mit 20 oder mehr Kohlenstoffatomen als "mehrfach ungesättigte Fettsäuren" oder "PUFAs" bezeichnet.

Nach der gängigen Nomenklatur werden PUFAs gemäß der Anzahl und Position der Doppelbindungen klassifiziert. Es gibt mehrere Reihen oder Familien von PUFAs abhängig von der Position der Doppelbindung, welche sich am dichtesten zum Methylende der Fettsäure befindet. Dabei sind zwei Reihen von besonderer nahrungsphysiologischer Bedeutung: die Omega-3-Reihe enthält eine Doppelbindung am dritten Kohlenstoffatom, während die Omega-6-Reihe bis zum sechsten Kohlenstoffatom keine Doppelbindung aufweist. Somit hat Docosahexaensäure ("DHA") eine Kettenlänge von 22 Kohlenstoffatomen mit 6 Doppelbindungen beginnend mit dem dritten Kohlenstoffatom vom Methylende und wird als "22:6 n-3" (all-cis-4,7,10,13,16,19-Docosahexaensäure) bezeichnet. Eine andere wichtige Omega-3-Fettsäure ist Eicosapentaensäure ("EPA"), die als "20:5 n-3" (all-*cis*-5,8,11,14,17-Eicosapentaensäure) bezeichnet wird. Eine wichtige Omega-6-Fettsäure ist Arachidonsäure ("ARA"), die als "20:4 n-6" (all-*cis*-5,8,11,14-Eicosatetraensäure) bezeichnet wird.

Bekannt sind Salze mehrfach ungesättigter Omega-3-Fettsäuren mit basischen Aminosäuren, wie sie beispielsweise in EP 0734373 B1 beschrieben werden. L-Lysinsalze von Omega-3-Fettsäuren haben sich gegenüber den freien Omega-3-Fettsäuren sowie den Alkali- und Erdalkalimetallsalzen von Omega-3-Fettsäuren als vergleichsweise oxidationsstabil erwiesen. Alkali- und Erdalkalimetallsalze von Omega-3-Fettsäuren sowie deren Herstellung werden beispielsweise in EP 1 260 496 A1 beschrieben.

L-Lysinsalze von Omega-3-Fettsäurewerden werden bisher dadurch hergestellt, dass die freien Omega-3-Fettsäuren mit L-Lysin in einem polaren Lösungsmittel oder Lösungsmittelgemisch umgesetzt und die erhaltenen Mischungen zur Trockne eingedampft werden. Als Lösungsmittel oder Lösungsmittelgemisch werden Wasser oder Wasser-Alkoholgemische verwendet. Die Omega-3-Fettsäure-L-Lysin-Salze werden dabei entweder als Feststoffe mit wachsartiger Konsistenz, so beschrieben in EP 0734373 B1, oder als kristalline Substanzen mit Schmelzpunkten unter Zersetzung von ca. 190°C, so beschrieben in DE 39 07 649 C2, erhalten.

Das Eindampfen von Lösungen der Omega-3-Fettsäure-L-Lysin-Salze in Wasser bzw. Wasser-Alkoholgemischen im Vakuum ist ein sehr energieintensiver Prozess. Darüber hinaus ist das Verfahren technisch sehr schwierig zu bewerkstelligen, da die Omega-3-Fettsäure-L-LysinSalze tensidische Eigenschaften besitzen und Lösungen davon beim Eindampfen zum Schäumen neigen: Beim Eindampfen nimmt die Viskosität der Lösungen stark zu, bis zähe Gele erhalten werden, die beim weiteren Trocknen feste Schäume bilden.

Eine weitere Möglichkeit zur Isolierung der Omega-3-Fettsäure-L-Lysin-Salze besteht in der Sprühtrocknung wässrig-ethanolischer Lösungen mit Konzentrationen an Omega-3-Fettsäure-L-Lysin-Salzen von > 50 Gew.-%. Jedoch erfordert der Einsatz eines organischen Lösungsmittels bei der Sprühtrocknung besondere Sicherheitsmaßnahmen, wie z.B. eine Inertgas-Atmosphäre. Auch durch die erforderliche Kondensation des Lösungsmittels werden die Kosten deutlich erhöht.

Es besteht daher nach wie vor ein Bedarf an möglichst einfachen und effektiven Verfahren zur Herstellung von Omega-3-Fettsäure-L-Lysin-Salzen, die sowohl energie- als auch kosteneffizient und mit Standardapparaturen ohne besondere Sicherheitsmaßnahmen durchführbar sind, und die die Omega-3-Fettsäure-L-Lysin-Salze in der für eine Anwendung als Lebensmittelzusatzstoffe erforderlichen Reinheit liefern.

Überraschenderweise wurde nun gefunden, dass Omega-3-Fettsäure-L-Lysin-Salze einfach dadurch erhalten werden können, dass eine Dispersion enthaltend eine oder mehrere Omega-3-Fettsäure(n), L-Lysin und 3 Gew.-% bis 15 Gew.-% Wasser, bezogen auf das Gesamtgewicht der Dispersion, bei einer Temperatur von 130 °C oder weniger geknetet wird.

Gegenstand der vorliegenden Erfindung ist demnach in einem ersten Aspekt ein Verfahren zur Herstellung einer Zusammensetzung enthaltend ein oder mehrere Omega-3-Fettsäure-L-Lysin-Salz(e), dadurch gekennzeichnet, dass eine Dispersion enthaltend eine oder mehrere Omega-3-Fettsäure(n), L-Lysin und 3 Gew.-% bis 15 Gew.-% Wasser, bezogen auf das Gesamtgewicht der Dispersion, bei einer Temperatur von 130 °C oder weniger so lange geknetet wird, bis 90 Gew.-% oder mehr der Omega-3-Fettsäure(n) zu Omega-3-Fettsäure-L-Lysin-Salz(en) umgewandelt sind.

Im Rahmen der vorliegenden Erfindung wird bei der Berechnung des Wassergehalts eventuell vorhandenes Kristallwasser im L-Lysin mit berücksichtigt.

Unter dem Begriff "kneten" wird im Rahmen der vorliegenden Erfindung in Übereinstimmung mit der Definition im "Duden" verstanden, dass die Dispersion "drückend verarbeitet" wird.

Im einfachsten Fall, beispielsweise im Laborbetrieb, kann das Kneten der Dispersion durch Einsatz eines Mixers erfolgen. Bei technischen Anwendungen kann das Kneten der Dispersion beispielsweise mittels eines Extruders bewerkstelligt werden. Damit ist es ebenfalls möglich, das hier beschriebene erfindungsgemäße Verfahren kontinuierlich durchzuführen.

Der Umsetzungsgrad der Reaktion kann dabei quantitativ mittels IR-Spektroskopie bestimmt werden, und zwar an Hand der Verschiebung der C=O-Absorptionsbande von etwa 1707 cm$^{-1}$ für freie Omega-3-Fettsäure(n) zu etwa 1578 cm$^{-1}$ nach Salzbildung. Einer Umwandlung von 90 Gew.-% oder mehr der Omega-3-Fettsäure(n) zu Omega-3-Fettsäure-L-Lysin-Salz(en) entspricht damit eine Verkleinerung der C=O-Absorptionsbande von etwa 1707 cm$^{-1}$ für freie Omega-3-Fettsäure(n) auf 10 % oder weniger. Eine geeignete Software, die die quantitative Auswertung von IR-Spektren ermöglicht, ist beispielsweise das Programm Opus Version 7.5 der Firma Bruker Optics.

Die Zeit, die jeweils benötigt wird, bis 90 Gew.-% oder mehr der Omega-3-Fettsäure(n) zu Omega-3-Fettsäure-L-Lysin-Salz(en) umgewandelt sind, steht in wechselseitigem Zusammenhang mit der eingesetzten Menge an Wasser sowie mit der eingestellten Temperatur.

Wie bereits eingangs erwähnt sind Omega-3-Fettsäuren oxidations- und temperaturempfindlich. Als Maßstab für die oxidative Zersetzung werden üblicherweise die Anisidinzahl (AZ) und die Peroxidzahl (PZ) bestimmt. Für die thermische Zersetzung ist der per Gelchromatographie bestimmbare Oligomerengehalt ein geeignetes Maß.

Die Peroxidzahl (PV) ist ein Maß für primäre Oxidationsprodukte (Hydroperoxidbildung an Doppelbindungen), und die Anisidinzahl (AV) ist ein Maß für sekundäre Zersetzungsprodukte (Carbonylverbindungen). Die Totoxzahl wird berechnet als Totox = 2*PV + AV (wobei PV in Milliäquivalent $O_2$ pro kg der Probe angegeben wird). Verfahren zur Bestimmung der Peroxidzahl (PV) und der Anisidinzahl (AV) werden in der Literatur beschrieben, vgl. z. B. "Official Methods and Recommended Practices of the AOCS", 6. Auflage 2013, herausgegeben von David Firestone, ISBN 978-1-893997-74-5. PV kann auch gemäß Ph. Eur. 2.5.5 (01/2008:20505) bestimmt werden, und AV kann auch gemäß Ph. Eur. 2.5.36 (0172008:20536) bestimmt werden.

Ein beispielhaftes Verfahren zur Bestimmung der Peroxidzahl (PV) einer Probe wird folgendermaßen durchgeführt:

Reagenzien und Lösung:

1. Essigsäure-Chloroform-Lösung (7,2 ml Essigsäure und 4,8 ml Chloroform).

2. Gesättigte Kaliumiodidlösung. Lichtgeschützt aufbewahren.

3. Natriumthiosulfatlösung, 0.1 N. Im Handel erhältlich.

4. 1 % Stärkelösung. Im Handel erhältlich.

5. Destilliertes oder entionisiertes Wasser.

Verfahren:

Führen Sie eine Blindwertbestimmung der Reagenzien durch.

1. 2,00 (±0,02) g der Probe in einem 100-ml-Erlenmeyerkolben mit Glasschliffstopfen abwiegen. Das Gewicht bis auf 0,01 g genau bestimmen.

2. Mit dem Messzylinder 12 ml der Essigsäure-Chloroform-Lösung hinzugeben.

3. Den Erlenmeyerkolben schwenken, bis die Probe vollständig gelöst ist (ggfs. ist vorsichtiges Erwärmen auf einer Heizplatte nötig).

4. Mit einer Messpipette 0,2 ml gesättige Kaliumiodidlösung hinzufügen.

5. Den Erlenmeyerkolben mit dem Stopfen verschließen und den Inhalt des Kolbens für exakt eine Minute schwenken.

6. Unverzüglich mit dem Messzylinder 12 ml destilliertes oder entionisiertes Wasser hinzugeben, den Kolben mit dem Stopfen verschließen und kräftig schütteln, um das Iod von der Chloroformschicht abzuscheiden.

7. 0,1 N Natriumthiosulfat in eine Bürette füllen.

8. Falls die Ausgangsfarbe der Lösung tief rot-orange ist, langsam unter Mischen titrieren, bis die Farbe heller wird. Wenn die Lösung am Anfang hell bernsteinfarben ist, zum Schritt 9 gehen.

9. Mit einer Dosiervorrichtung 1 ml Stärkelösung als Indikator hinzugeben.

10. Titrieren, bis die blaugraue Farbe in der wässrigen Phase (obere Schicht) verschwindet.

11. Den genauen ml-Wert des Titriermittels bis auf zwei Dezimalstellen festhalten.

Berechnung:

S = Titration der Probe

B = Blindtitration

Peroxidzahl = (S - B) * N Thiosulfat * 1.000 / Gewicht der Probe

Ein beispielhaftes Verfahren zur Bestimmung der Anisidinzahl (AV) einer Probe wird folgendermaßen durchgeführt:

Die Anisidinzahl ist definiert als das 100fache der optischen Dichte gemessen in einer 1cm-Zelle einer Lösung enthaltend 1 g der zu untersuchenden Substanz in 100 ml einer Mischung aus Lösungsmitteln und Reagenzien gemäß der folgenden Methode. Die Arbeitsabläufe müssen so rasch wie möglich ausgeführt werden, wobei die Einwirkung von aktinischem Licht vermieden werden soll.

Probelösung (a): 0,500 g der zu untersuchenden Lösung in Trimethylpentan auflösen und mit dem gleichen Lösungsmittel auf 25,0 ml verdünnen.

Probelösung (b): Zu 5,0 ml der Probelösung (a) 1,0 ml einer 2,5g/l-Lösung von p-Anisidin in Eisessig geben, schütteln und vor Licht geschützt lagern.

Vergleichslösung: Zu 5,0 ml Trimethylpentan 1,0 ml einer 2,5g/l-Lösung von p-Anisidin in Eisessig geben, schütteln und vor Licht geschützt lagern. Den Absorptionsgrad der Probelösung (a) bei maximal 350 nm messen, wobei Trimethylpentan als Ausgleichsflüssigkeit benutzt wird. Nach exakt 10 Minuten nach Herstellung den Absorptionsgrad der Probelösung (b) bei 350 nm messen, wobei die Vergleichslösung als Ausgleichsflüssigkeit benutzt wird. Die Anisidinzahl (AV) wird aus der folgenden Gleichung errechnet:

$$AV = (25*(1{,}2*A1 - A2))/m$$

A1 = Absorptionsgrad der Probelösung (b) bei 350 nm,

A2 = Absorptionsgrad der Probelösung (a) bei 350 nm,

m = Masse der zu untersuchenden Substanz in der Probelösung (a) in Gramm.

Wenn die Stabilität der Proben gegenüber Oxidation verglichen wird, indem (1) der Oxidationsgrad gemessen wird, (2) die Proben oxidierenden Bedingungen ausgesetzt werden und (3) erneut der Oxidationsgrad gemessen wird, wird der Oxidationsgrad im Kontext der vorliegenden Erfindung in den Schritten (1) und (3) vorzugsweise bewertet, indem die Peroxidzahl (PV) und/oder die Anisidinzahl (AV) bestimmt wird; ferner werden die oxidierenden Bedingungen in Schritt (2) vorzugsweise aus einer der folgenden ausgewählt: Lagerung in offenen Containern, die über einen vorbestimmten Zeitraum von mindestens zehn Tagen Luft bei Raumtemperatur ausgesetzt sind; Lagerung in offenen Containern, die über einen vorbestimmten Zeitraum von mindestens drei Tagen Luft bei 50 °C ausgesetzt sind.

Überraschenderweise wurde gefunden, dass die Oxidationsempfindlichkeit der erfindungsgemäß hergestellten Omega-3-Fettsäure-L-Lysin-Salze nicht nur von der im Herstellungsverfahren eingestellten Temperatur, sondern auch von dem im Herstellungsverfahren eingesetzten Wassergehalt abhängt.

In einer bevorzugten Ausgestaltung des erfindungsgemäßen Verfahrens enthält die eingesetzte Dispersion 5 Gew.-% bis 10 Gew.-% Wasser, vorzugsweise 7 Gew.-% bis 8 Gew.-% Wasser, bezogen auf das Gesamtgewicht der Dispersion.

In einer weiteren bevorzugten Ausgestaltung des erfindungsgemäßen Verfahrens liegt die eingestellte Temperatur im Bereich von 25 °C bis 100 °C, vorzugsweise im Bereich von 40 °C bis 80 °C.

In bevorzugten Aspekten der vorliegenden Erfindung enthalten bevorzugte Ausgangsdispersionen signifikante Mengen an freien Fettsäuren. Dementsprechend beträgt die Gesamtmenge an Fettsäuren (d. h. Fettsäuren mit freien Carboxylgruppen) vorzugsweise mindestens x Gewichtsprozent der Ausgangsdispersion, ohne dabei die flüchtigen Bestandteile zu berücksichtigen, wobei x ausgewählt ist aus 30, 40, 50, 60, 65.

In bevorzugen Aspekten der vorliegenden Erfindung enthalten bevorzugte Ausgangsdispersionen signifikante Mengen an L-Lysin. Dementsprechend beträgt die Gesamtmenge an L-Lysin vorzugsweise mindestens y Gewichtsprozent der Ausgangsdispersion, ohne dabei die flüchtigen Bestandteile zu berücksichtigen, wobei y ausgewählt ist aus 15, 20, 25, 30.

Omega-3-Fettsäuren, die in dem erfindungsgemäßen Verfahren einzeln oder in beliebiger Kombination verwendet werden können, umfassen beispielsweise Eicosatriensäure (ETE) 20:3 (n-3) (all-*cis*-11,14,17-Eicosatriensäure), Eicosatetraensäure (ETA) 20:4 (n-3) (all-cis-8,11,14,17-Eicosatetraensäure), Heneicosapentaensäure (HPA) 21:5 (n-3) (all-cis-6,9,12,15,18-Heneicosapentaensäure), Docosapentaensäure (Clupanodonsäure) (DPA) 22:5 (n-3) (all-cis-7,10,13,16,19-Docosapentaensäure, Tetracosapentaensäure 24:5 (n-3) (all-cis-9,12,15,18,21-Tetracosapentaensäure), Tetracosahexaensäure (Nisinsäure) 24:6 (n-3) (all-cis-6,9,12,15,18,21-Tetracosahexaensäure).

Mehrfach ungesättigte Omega-3-Fettsäuren, die in dem erfindungsgemäßen Verfahren eingesetzt werden können, können aus jedem geeigneten Ausgangsstoff gewonnen werden, der zusätzlich mit jedem geeigneten Verfahren bearbeitet werden kann. Zu den typischen Ausgangsstoffen zählen alle Teile von Fischkarkassen, Gemüse und anderen Pflanzen als auch Material aus mikrobieller Fermentation oder Fermentation von Algen. Typische Verarbeitungsmethoden für solche Ausgangsstoffe sind u. a. Schritte zur Rohölgewinnung, wie Extraktion und Abtrennung von den Ausgangsstoffen, als auch Schritte zur Veredlung von Rohölen, wie Absetzen und Degummierung, Entsäuerung, Bleichen und Geruchsbeseitigung (vgl. z. B. "EFSA Scientific Opinion on Fish Oil for Human Consumption"). Zu weiteren Verarbeitungsverfahren gehören u. a. Schritte zur zumindest teilweisen Umwandlung von Omega-3-Fettsäureestern in die entsprechenden freien Omega-3-Fettsäuren oder deren anorganische Salze.

Mehrfach ungesättigte Omega-3-Fettsäuren, die in dem erfindungsgemäßen Verfahren eingesetzt werden können, lassen sich auch durch Spaltung der Omega-3-Fettsäureestern und anschließende Entfernung der zuvor als Ester gebundenen Alkohole aus Zusammensetzungen gewinnen, die hauptsächlich aus Omega-3-Fettsäureestern bestehen. Vorzugsweise wird die Esterspaltung unter basischen Bedingungen durchgeführt. Verfahren zur Esterspaltung sind aus dem Stand der Technik wohlbekannt.

Im Rahmen der vorliegenden Erfindung bevorzugt einzusetzende Omega-3-Fettsäuren sind Eicosapentaensäure ("EPA") und Docosahexaensäure ("DHA").

In einer bevorzugten Ausgestaltung des erfindungsgemäßen Verfahrens wird eine Mischung aus Omega-3-Fettsäuren eingesetzt, die in Summe 30 Gew.-% oder mehr, vorzugsweise 50 Gew.-% oder mehr, besonders bevorzugt 70 Gew.-% oder mehr, ganz besonders bevorzugt 90 Gew.-% oder mehr, Eicosapentaensäure und Docosahexaensäure, bezogen auf das Gesamtgewicht der Fettsäuren, enthält.

Das Molverhältnis M der Summe aller Carboxylgruppen der Fettsäuren zu der Summe aller Lysinmoleküle sollte möglichst äquimolar sein, um eine möglichst quantitative Salzbildung zu ermöglichen.

In einer bevorzugten Ausgestaltung des erfindungsgemäßen Verfahrens liegt das Molverhältnis M der Summe aller Carboxylgruppen der Fettsäuren zu der Summe aller Lysinmoleküle im Bereich $0.8 \le M \le 1.2$, weiter bevorzugt im Bereich $0.9 \le M \le 1.1$, noch weiter bevorzugt im Bereich $0.95 \le M \le 1.05$, besonders bevorzugt im Bereich $0.98 \le M \le 1.02$.

Überraschenderweise lassen sich die erfindungsgemäß hergestellten Omega-3-Fettsäure-L-LysinSalze problemlos trocknen.

In einer bevorzugten Ausgestaltung des erfindungsgemäßen Verfahrens wird daher die erhaltene Zusammensetzung, vorzugsweise in einem Temperaturbereich von 50 °C bis 60 °C und bei einem Druck von 20 mbar oder weniger, getrocknet, bis ein Feststoff erhalten wird. Vorzugsweise weist der erhaltene Feststoff einen Wassergehalt von ≤ 2 Gew.-%, besonders bevorzugt von ≤ 1 Gew.-%, gemessen durch Karl-Fischer-Titration auf.

Überraschenderweise wurde darüber hinaus gefunden, dass die erfindungsgemäß hergestellten Omega-3-Fettsäure-L-Lysin-Salze sehr spröde sind und sich mit gängigen Mühlen, beispielsweise einer Retsch Kugelmühle, einfach mahlen lassen, obwohl dies aufgrund der früher beschriebenen Eigenschaften der Omega-3-Fettsäure-L-Lysin-Salze nicht zwangsläufig zu erwarten war.

In einer bevorzugten Ausgestaltung des erfindungsgemäßen Verfahrens wird daher der erhaltene Feststoff vermahlen.

Eine erfindungsgemäße Zusammensetzung enthaltend 90 Gew.-% oder mehr Omega-3-Fettsäure-L-Lysin-Salz(e) weist typischerweise eine Korngröße $D_{90}$ von 100 $\mu$m oder mehr und eine Korngröße $D_{50}$ von 17 $\mu$m oder mehr und eine Korngröße $D_{10}$ von 5 $\mu$m oder mehr auf, wenn die Zusammensetzung für einen Zeitraum von 30 Minuten in einer Retsch-Kugelmühle Typ SM1 mit einer Mahlgeschwindigkeit von 250 Umdrehungen pro Minute und einem Durchmesser der Mahlkugeln von 30 mm vermahlen wurde.

Gegenstand der vorliegenden Erfindung ist damit auch eine Zusammensetzung enthaltend 90 Gew.-% oder mehr Omega-3-Fettsäure-L-Lysin-Salz(e), dadurch gekennzeichnet, dass die Zusammensetzung eine Korngröße $d_{90}$ von 100 $\mu$m oder mehr, vorzugsweise 120 $\mu$m oder mehr, besonders bevorzugt 140 $\mu$m oder mehr, und eine Korngröße $d_{50}$ von 17 $\mu$m oder mehr, vorzugsweise 19 $\mu$m oder mehr, besonders bevorzugt 21 $\mu$m oder mehr, und eine Korngröße $d_{10}$ von 5 $\mu$m oder mehr, vorzugsweise 6 $\mu$m oder mehr, besonders bevorzugt 7 $\mu$m oder mehr, aufweist, wenn die Zusammensetzung für einen Zeitraum von 30 Minuten in einer Retsch-Kugelmühle Typ SM1 mit einer Mahlgeschwindigkeit von 250 Umdrehungen pro Minute und einem Durchmesser der Mahlkugeln von 30 mm vermahlen wurde.

Gegenstand der vorliegenden Erfindung ist in einem weiteren Aspekt eine Zusammensetzung erhältlich oder erhalten durch das hier beschriebene erfindungsgemäße Verfahren. Dementsprechend ist auch Gegenstand der vorliegenden Erfindung eine Zusammensetzung erhältlich oder erhalten durch ein Verfahren, welches dadurch gekennzeichnet ist, dass eine Dispersion enthaltend eine oder mehrere Omega-3-Fettsäure(n), L-Lysin und 3 Gew.-% bis 15 Gew.-% Wasser, bezogen auf das Gesamtgewicht der Dispersion, bei einer Temperatur von 130 °C oder weniger so lange geknetet wird, bis 90 Gew.-% oder mehr der Omega-3-Fettsäure(n) zu Omega-3-Fettsäure-L-Lysin-Salz(en) umgewandelt sind.

Gegenstand der vorliegenden Erfindung ist außerdem die Verwendung einer erfindungsgemäßen Zusammensetzung für die Herstellung von Nahrungsmitteln, Nahrungsergänzungsmitteln oder pharmazeutischen Produkten.

Im Rahmen der vorliegenden Erfindung umfassen Nahrungs- und Nahrungsergänzungsmittel, ohne darauf beschränkt zu sein, Backwaren, Vitaminzusätze, Getränkepulver, Knetteige, Rührteige, gebackene Nahrungsmittel wie z. B. Kuchen, Käsekuchen, Pies, Cupcakes, Kekse, Brote, Brötchen, Plätzchen, Muffins, Feingebäck, Teegebäck und Croutons; flüssige Nahrungsmittelprodukte wie z. B. Getränke, Energydrinks, Säuglingsanfangsnahrung, Trinkmahlzeiten, Fruchtsäfte, Multivitaminsirupe, Mahlzeitersatz; halbfeste Nahrungsmittelprodukte wie Babynahrung, Joghurt, Käse, Getreideflocken, Pfannkuchenmischungen; Nährriegel wie Energieriegel; verarbeitetes Fleisch; Eiscremes; gefrorene Desserts; gefrorene Joghurts; Waffelmischungen; Salatdressings; Eiersatzmischungen; Cookies, Crackers, Süßwaren, Snacks, Müesli-/Snackriegel, Toastergebäck, gesalzenes Knabbergebäck wie Kartoffelchips, Maischips, Tortillachips, extrudierte Snacks, Popcorn, und Nüsse; besondere Snacks wie Dips, getrocknete Früchte, Fleischsnacks, frittierte Snacks, Gesundheitsriegel und Reis-/Maiswaffeln; Süßwaren wie Bonbons; Instantnahrungsmittel wie Instantnudeln, Instantsuppenwürfel oder Instantpulver.

Im Rahmen der vorliegenden Erfindung können pharmazeutische Produkte neben den hier beschriebenen Omega-3-Fettsäure-L-Lysin-Salzen sowohl pharmazeutisch zulässige Hilfsstoffe als auch pharmazeutische Wirkstoffe wie z. B. Statine, blutdrucksenkende Mittel, Antidiabetika, Antidementiva, Antidepressiva, Mittel gegen Fettleibigkeit, Appetitzügler und Mittel zur Verbesserung des Gedächtnisses und/oder der kognitiven Funktionen, enthalten.

Wie bereits festgestellt sind Salze aus basischen Aminosäuren und mehrfach ungesättigten Fettsäuren aus dem Stand der Technik bekannt (vgl. EP 0734373 B1), wobei es unbekannt war, dass Salze von L-Lysin und mehrfach ungesättigten Fettsäuren durch Kneten einer Dispersion aus mehrfach ungesättigten Fettsäuren, L-Lysin und Wasser hergestellt werden können. Hierbei ist wesentlich, dass Salze aus basischen Aminosäuren und mehrfach ungesättigten Fettsäuren bisher als "sehr dicke, transparente Öle, die sich bei niedrigen Temperaturen in Feststoffe mit wachsartigem Aussehen und wachsartiger Beschaffenheit umwandeln" (vgl. EP 0734373 B1, S. 1, Z. 47-48) beschrieben wurden. Infolgedessen hätte ein Fachmann nicht erwarten können, dass Omega-3-Fettsäure-L-Lysin-Salzen durch Kneten einer Dispersion von Omega-3-Fettsäuren, L-Lysin und Wasser hergestellt werden können. Es war somit überraschend, dass im Rahmen der vorliegenden Erfindung herausgefunden wurde, dass Omega-3-Fettsäure-L-Lysin-Salze in der Tat durch Kneten mühelos gewonnen werden können. Wie bereits eingangs beschrieben sind die Knetbedingungen an die jeweils eingestellte Temperatur und die eingesetzte Wassermenge anzupassen. Solche Anpassungen vorzunehmen gehört jedoch zur routinemäßigen Laborarbeit eines Durchschnittsfachmanns.

Das erfindungsgemäße Verfahren wird hier im Detail zur Anwendung mit Omega-3-Fettsäuren beschrieben. Der Einsatz von Omega-6-Fettsäuren ist jedoch gleichfalls möglich.

Omega-6-Fettsäuren, die in dem erfindungsgemäßen Verfahren einzeln oder in beliebiger Kombination verwendet werden können, umfassen beispielsweise Eicosadiensäure 20:2 (n-6) (all-cis-11,14-Eicosadiensäure), Dihomogammalinolensäure (DGLA) 20:3 (n-6) (all-cis-8,11,14-Eicosatriensäure), Arachidonsäure (ARA) 20:4 (n-6) (all-cis-5,8,11,14-Eicosatetraensäure), Docosadiensäure 22:2 (n-6) (all-cis-13-16-Docosadiensäure), Adrensäure 22:4 (n-6) (all-cis-7,10,13,16-Docosatetraensäure), Docosapentaensäure (Osbondsäure) 22:5 (n-6) (all-cis-4,7,10,13,16-Docosapentaensäure), Tretracosatetraensäure 24:4 (n-6) (all-cis-9,12,15,18-Tetracosatetraensäure), Tetracosapentaensäure 24:5 (n-6) (all-cis-6,9,12,15,18-Tetracosapentaensäure).

Die vorliegende Erfindung wird anhand der nachfolgenden nicht beschränkenden Versuche näher beschrieben.

**Versuche:**

Analytische Methoden:

Primäre Oxidationsprodukte (Hydroperoxide an Doppelbindungen) wurden durch die Ermittlung der Peroxidzahl (PV) gemäß Ph. Eur. 2.5.5 (01/2008:20505) mengenmäßig bestimmt. Sekundäre Oxidationsprodukte (Carbonylverbindungen) wurden durch die Ermittlung der Anisidinzahl (AV) gemäß Ph. Eur. 2.5.36 (01/2008:20536) mengenmäßig bestimmt.

Oligomere Omega-3-Fettsäure-Bestandteile sowie ihre Derivate (zusammenfassend als Oligomerengehalt bezeichnet) wurden gelchromatographisch quantifiziert (GPC, Styrol-Divinylbenzol-Phase mit Tetrahydrofuran enthaltend Trifluoressigsäure als Eluent). Für die Bestimmung wurde ein Brechungsindexdetektor (RI) benutzt. Weil spezifische Reaktionsfaktoren für die Bestandteile der Proben nicht bekannt waren, wurden die Mengenverhältnisse auf der Grundlage von Brechungsverhältnissen der gesamten Fläche des Chromatogramms errechnet.

Der Wassergehalt wurde durch Karl-Fischer-Titration bestimmt.

Säurewerte wurden durch Titration mit Kaliumhydroxid bestimmt.

**1. Versuch mit 3,8 % Wasserzusatz**

270,0 g Fettsäure (hergestellt aus einem Fischöl mit Fettsäuregehalten von 50 % EPA und 20 % DHA) mit einer Säurezahl von 178,85 mg KOH/g und 141,4 g Lysin-Monohydrat (dies entspricht 125,9 g L-Lysin und 15,5 g Wasser) wurden im Kneter (Thermo-Fisher Rheomix 3000E, Volumen ca. 310 ml mit Walzenrotoren (R3)) vorgelegt und anschließend zunächst bei 70 - 90 °C geknetet. Da sich zunächst keine homogene Mischung bildete, wurde die Temperatur auf 120 °C erhöht. Es bildete sich eine homogene Schmelze, aus der Wasserdampf in Form von Bläschen ausgaste.

Nach ca. 10 min bei 120 °C wurde die Mischung aus dem Kneter ausgetragen. Man erhielt 339 g eines Produktes, das beim Erkalten erstarrte. 294,2 g des Feuchtprodukts wurden über Nacht im Trockenschrank bei 50 - 60 °C und 20 mbar getrocknet. Nach Zerkleinern erhielt man 283 g eines Feststoffes mit einer Schüttdichte von 0,665g/ml.

**2. Versuch mit 7,5 % Wasserzusatz**

250,0 g Fettsäure (hergestellt aus einem Fischöl mit Fettsäuregehalten von 50 %EPA/20 % DHA) mit einer Säurezahl von 178,85 mg KOH und 131,0 g Lysin Monohydrat (dies entspricht 116,6 g L-Lysin und 14,4 g Wasser) wurden unter Zusatz von 15,3 g Wasser analog Versuch 1 für 10 min bei 50°C umgesetzt. Man erhielt 376 g Feuchtprodukt als plastischen Feststoff. 335,8 g des Feuchtprodukts wurden über Nacht im Trockenschrank bei 50 - 60 °C und 20 mbar getrocknet. Nach Zerkleinern erhielt man 313 g eines Feststoffes mit einer Schüttdichte von 0,585 g/ml.

**3. Versuch mit 15 % Wasserzusatz**

230,0 g Fettsäure (hergestellt aus einem Fischöl mit Fettsäuregehalten von 50 %EPA/20 % DHA) mit einer Säurezahl von 175,3 mg KOH und 117,8 g Lysin-Monohydrat (dies entspricht 104,9 g L-Lysin und 12,9 g Wasser) wurden unter Zusatz von 46,2 g Wasser analog Versuch 1 für 60 min bei 25°C umgesetzt. Man erhielt 373 g Feuchtprodukt als Creme. 333 g des Feuchtprodukts wurden über Nacht im Trockenschrank bei 50 - 60 °C und 20 mbar getrocknet, wobei ein fester Schaum entstand. Nach Zerkleinern erhielt man 282 g eines Feststoffes mit einer Schüttdichte von 0,375 g/ml.

**4. Vergleichsversuch mit 20,4 % Wasserzusatz**

200,0 g Fettsäure (hergestellt aus einem Fischöl mit Fettsäuregehalten von 50 %EPA/20 % DHA) mit einer Säurezahl von 175,3 mg KOH und 102,5 g Lysin-Monohydrat (dies entspricht 91,2 L-Lysin und 11,3 g Wasser) wurden unter Zusatz von 63,5 g Wasser analog Versuch 1 für 60 min bei 25 °C umgesetzt. Man erhielt 371 g Feuchtprodukt als klebrige Paste. 330 g des Feuchtprodukts wurden über Nacht im Trockenschrank bei 50 - 60 °C und 20 mbar getrocknet, wobei ein fester Schaum entstand. Nach Zerkleinern erhielt man 265 g eines Feststoffes mit einer Schüttdichte von 0,347 g/ml.

**5. Vergleichsversuch ohne Wasserzusatz**

250,0 g Fettsäure (hergestellt aus einem Fischöl mit Fettsäuregehalten von 50 %EPA/20 % DHA) mit einer Säurezahl von 175,3 mg KOH/g und 114 g wasserfreies Lysin wurden im Kneter (Thermo-Fisher Rheomix 3000E, Volumen ca. 310 ml mit Walzenrotoren (R3)) vorgelegt und anschließend zunächst bei 100 °C geknetet. Da sich zunächst keine homogene Mischung bildete, wurde die Temperatur schrittweise auf 150 °C erhöht. Nach 20 min bei 150 °C wurde der Versuch abgebrochen und die Mischung aus dem Kneter ausgetragen. Man erhielt 330 g eines Produktes, bei dem noch sehr deutliche Einschlüsse von festem Lysin zu erkennen waren.

Die Bedingungen der Knetversuche im Thermo-Fisher Rheomix 3000E sowie die Wassergehalte nach Trocknung bei 50-60 °C/20 mbar sind in Tabelle 1 zusammengefasst. Bei den Versuchen wurden die Produkte nach Abkühlen mechanisch aus dem Kneter herausgekratzt.

Tabelle 1:

| Versuch Nr. | Wassergehalt [Gew. %] | Temperatur [°C] | Zeit [min] | Wassergehalt nach Trocknung [Gew. %] | Schüttdichte [g/ml] |
|---|---|---|---|---|---|
| 5 | 0,0 | 150 | 20 | 0,63 | n. b. |
| 1 | 3,8 | 120 | 10 | 0,43 | 0,665 |
| 2 | 7,5 | 55 | 10 | 0,22 | 0,585 |
| 3 | 15,0 | 25 | 60 | 0,20 | 0,375 |
| 4 | 20,4 | 22 | 60 | 0,31 | 0,347 |

Während mit 20,4 % und 15,0 % Wasser bereits durch Kneten bei Raumtemperatur eine homogene Mischung erhalten wurde, mussten bei den Versuchen mit 7,5 % und 3,8 % (dies entspricht dem Einsatz von Lysin-Monohydrat) dafür Temperaturen von 55 °C und 130 °C angewandt werden. Beim Versuch ohne Wasserzusatz wurde selbst nach 20 min bei 150 °C keine homogene Mischung erreicht. Im Produkt waren danach immer noch gewisse Mengen festen Lysins erkennbar.

Während die Produkte mit 0 %, 3,8 % und 7,5 % Wasser beim Trocknen bei 50-60 °C im Vakuum ihre Form nicht merkbar veränderten, bildeten die Produkte mit 15 % und 20,4 % Wasser feste Schäume, die sich aber leicht zerkleinern ließen. Die Schaumbildung führt jedoch zu einer deutlichen Reduktion der Schüttdichten der zerkleinerten getrockneten Feststoffe (Tabelle 2). Während diese mit 3,8 % und 7,5 % Wasser bei 0,665 g/ml bzw. 0,585 g/ml lagen, wurden mit 15 % und 20,4 % Wasser nur Schüttdichten von 0,375 g/ml bzw. 0,347 g/ml erhalten. Nach dem Mahlen war aber kein signifikanter Unterschied mehr feststellbar. In allen Fällen wurden Restwassergehalte von unter 0,5 % erreicht.

Die Anisidin- und Peroxidzahlen (AZ) und (PZ) sowie die Oligomerengehalte der Produkte aus den Knetversuchen im Thermo-Fisher Rheomix 3000E sind in Tabelle 2 zusammengefasst.

Tabelle 2:

| Versuch Nr. | WasserZusatz [Gew. %] | Temperatur [°C] | AZ | PZ | Totox-Zahl (2PZ+AZ) | Oligomere [Fl-%] |
|---|---|---|---|---|---|---|
| 5 | 0,0 | 150 | 7,2 | 4,0 | 15,1 | 5,3 |
| 1 | 3,8 | 120 | 26,1 | < 1 | 28,1 | 0,5 |
| 2 | 7,5 | 55 | 2,6 | < 1 | 4,6 | 0,2 |
| 3 | 15,0 | 25 | 3 | < 1 | 5 | 0,2 |

(fortgesetzt)

| Versuch Nr. | WasserZusatz [Gew. %] | Temperatur [°C] | AZ | PZ | Totox-Zahl (2PZ+AZ) | Oligomere [Fl-%] |
|---|---|---|---|---|---|---|
| 4 | 20,4 | 22 | 5,1 | < 1 | 7,1 | 0,2 |

Wie die von den Proben bestimmten Werte zeigen, steigt sowohl die TOTOX-Zahl als auch der Oligomerengehalt mit der Temperatur an. Während die Werte für die Probe mit 3,8 % Wasser noch akzeptabel sind, ist bei der Probe ohne Wasserzusatz sowohl die TOTOX-Zahl als auch der Oligomerengehalt deutlich angestiegen.

**6. Mahlversuche mit den Produkte aus den Versuchen 1 bis 5**

Je ca. 70 g der getrockneten Proben aus der Versuchen 1 bis 4 wurden in einer Retsch-Kugelmühle Typ SM1 für jeweils 30 min mit einer Mahlgeschwindigkeit von 250 Umdrehungen pro Minute und einem Durchmesser der Mahlkugeln von 30 mm vermahlen. Das Volumen des Mahlbechers betrug 250 ml, und sowohl Mahlbecher als auch Mahlkugeln waren aus Achat gefertigt. Die Korngrößenverteilungen der vermahlenen Produkte sind in Tabelle 3 zusammengefasst.

Tabelle 3:

| Versuch Nr. | d10 [μm] | d50 [μm] | d90 [μm] |
|---|---|---|---|
| 1 | 5,6 | 18,0 | 117,1 |
| 2 | 6,0 | 20,8 | 135,8 |
| 3 | 7,4 | 24,7 | 143,7 |
| 4 | 5,6 | 15,3 | 79,4 |

Mit der Standardkugelmühle konnten alle Produkte aus der Knetversuchung nach Trocknung auf mittlere Korngrößen von unter 50 μm vermahlen werden. Das aus dem Versuch ohne Wasserzusatz erhaltene Produkt, bei dem die Salzbildung nicht vollständig war, ergab in der Kugelmühle dünne Schuppen mit Durchmessern von ca. 5 mm, die einfach verbiegbar waren. Ein ähnliches Ergebnis ist für Produkte mit höheren Restwassergehalten zu erwarten.

**7. Mahlversuch mit Stiftmühle**

Ca. 200 g eines granulierten Produktes mit einer mittleren Korngröße von 200 μm wurden in einer Jehmlich Stiftmühle Rekord 224 bei 17.000 U/min vermahlen. Das Produkt konnte auf Korngrößen von $d_{90}$ 13,2 μm und $d_{50}$ von 3.2 μm vermahlen werden, ohne dass irgendwelche Anbackungen in der Mühle erkennbar waren.

**Patentansprüche**

1.   Verfahren zur Herstellung einer Zusammensetzung enthaltend ein oder mehrere Omega-3-Fettsäure-L-Lysin-Salz(e), **dadurch gekennzeichnet, dass** eine Dispersion enthaltend eine oder mehrere Omega-3-Fettsäure(n), L-Lysin und 3 Gew.-% bis 15 Gew.-% Wasser, bezogen auf das Gesamtgewicht der Dispersion, bei einer Temperatur von 130 °C oder weniger so lange geknetet wird, bis 90 Gew.-% oder mehr der Omega-3-Fettsäure(n) zu Omega-3-Fettsäure-L-Lysin-Salz(en) umgewandelt sind.

2.   Verfahren nach Anspruch 1, **dadurch gekennzeichnet, dass** die Dispersion 5 Gew.-% bis 10 Gew.-% Wasser, vorzugsweise 7 Gew.-% bis 8 Gew.-% Wasser, bezogen auf das Gesamtgewicht der Dispersion, enthält.

3.   Verfahren nach Anspruch 1 oder 2, **dadurch gekennzeichnet, dass** die Temperatur im Bereich von 25 °C bis 100 °C, vorzugsweise im Bereich von 40 °C bis 80 °C, liegt.

4.   Verfahren nach einem der Ansprüche 1 bis 3, **dadurch gekennzeichnet, dass** die Omega-3-Fettsäuren in Summe 30 Gew.-% oder mehr, vorzugsweise 50 Gew.-% oder mehr, besonders bevorzugt 70 Gew.-% oder mehr, ganz besonders bevorzugt 90 Gew.-% oder mehr, Eicosapentaensäure und Docosahexaensäure, bezogen auf das Gesamtgewicht der Fettsäuren, enthalten.

**5.** Verfahren nach einem der Ansprüche 1 bis 4, **dadurch gekennzeichnet, dass** das Molverhältnis M der Summe aller Carboxylgruppen der Fettsäuren zu der Summe aller Lysinmoleküle im Bereich $0.8 \leq M \leq 1.2$, vorzugsweise im Bereich $0.9 \leq M \leq 1.1$, weiter bevorzugt im Bereich $0.95 \leq M \leq 1.05$, besonders bevorzugt im Bereich $0.98 \leq M \leq 1.02$, liegt.

**6.** Verfahren nach einem der Ansprüche 1 bis 5, **dadurch gekennzeichnet, dass** die Zusammensetzung, vorzugsweise in einem Temperaturbereich von 50 °C bis 60 °C und bei einem Druck von 20 mbar oder weniger, getrocknet wird, bis ein Feststoff erhalten wird.

**7.** Verfahren nach Anspruch 6, **dadurch gekennzeichnet, dass** der Feststoff einen Wassergehalt von $\leq 2$ Gew.-%, vorzugsweise $\leq 1$ Gew.-%, gemessen durch Karl-Fischer-Titration aufweist.

**8.** Verfahren nach Anspruch 6 oder Anspruch 7, **dadurch gekennzeichnet, dass** der Feststoff vermahlen wird.

**9.** Zusammensetzung erhältlich oder erhalten durch ein Verfahren nach einem der Ansprüche 1 bis 8.

**10.** Zusammensetzung enthaltend 90 Gew.-% oder mehr Omega-3-Fettsäure-L-Lysin-Salz(e), **dadurch gekennzeichnet, dass** die Zusammensetzung eine Korngröße $d_{90}$ von 100 $\mu$m oder mehr und eine Korngröße $d_{50}$ von 17 $\mu$m oder mehr und eine Korngröße $d_{10}$ von 5 $\mu$m oder mehr aufweist, wenn die Zusammensetzung für einen Zeitraum von 30 Minuten in einer Retsch-Kugelmühle Typ SM1 mit einer Mahlgeschwindigkeit von 250 Umdrehungen pro Minute und einem Durchmesser der Mahlkugeln von 30 mm vermahlen wurde.

**11.** Verwendung einer Zusammensetzung nach Anspruch 9 oder 10 für die Herstellung von Nahrungsmitteln, Nahrungsergänzungsmitteln oder pharmazeutischen Produkten.

**Europäisches Patentamt**
**European Patent Office**
**Office européen des brevets**

# EUROPÄISCHER RECHERCHENBERICHT

Nummer der Anmeldung

EP 16 17 1275

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | KR 2003 0053484 A (LIPOZEN INC [KR]) 28. Juni 2003 (2003-06-28) * Zusammenfassung * * Absätze [0004], [0020] - [0029] * * Beispiele 14,7 * ----- | 1-11 | INV. A23D7/005 A61K31/202 C07C51/00 C07C57/03 C11C1/02 A23L33/17 |
| X | GB 2 216 522 A (BIOREX KFT [HU]) 11. Oktober 1989 (1989-10-11) * Ansprüche 1-16 * * Beispiele 1,4,5,19,20 * * Seiten 9-11 * ----- | 1-11 | |
| X | WO 2006/059169 A2 (SINNEX MUESZAKI FEJLESZTOE ES [HU]; SZILBEREKY JENOE [HU]; JEDNAKOVITS) 8. Juni 2006 (2006-06-08) | 9-11 | |
| A | * Ansprüche 1-7 * * Beispiele 1-4 * ----- | 1-8 | |
| X | GB 2 216 418 A (BIOREX KFT [HU]) 11. Oktober 1989 (1989-10-11) | 9-11 | |
| A | * Ansprüche 1-14 * * Beispiele 3,1,7,8,16 * ----- | 1-8 | RECHERCHIERTE SACHGEBIETE (IPC) |
| X | WO 95/16661 A1 (PROSPA BV [NL]; BRUZZESE TIBERIO [IT]) 22. Juni 1995 (1995-06-22) | 9-11 | A23L A23D A61K |
| A | * Ansprüche 1-8 * * Beispiele 3,4,7,8,9,12,14,15 * ----- | 1-8 | C07C C11C |
| A | JP H09 77725 A (AJINOMOTO KK) 25. März 1997 (1997-03-25) * Zusammenfassung * ----- | 1-11 | |
|  | -/-- | | |

Der vorliegende Recherchenbericht wurde für alle Patentansprüche erstellt

| Recherchenort | Abschlußdatum der Recherche | Prüfer |
|---|---|---|
| München | 1. Juli 2016 | Barac, Dominika |

Seite 1 von 2

Europäisches
Patentamt

European
Patent Office

Office européen
des brevets

# EUROPÄISCHER RECHERCHENBERICHT

**Nummer der Anmeldung**

EP 16 17 1275

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | BRUNO NOVALES ET AL: "Self-Assembly and Foaming Properties of Fatty Acid?Lysine Aqueous Dispersions", LANGMUIR,, Bd. 26, Nr. 8, 1. Januar 2010 (2010-01-01), Seiten 5329-5334, XP009190778, ISSN: 1520-5827 * das ganze Dokument *<br>----- | 1-11 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 1. Juli 2016 | Barac, Dominika |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
..................................................................
& : Mitglied der gleichen Patentfamilie, übereinstimmendes
    Dokument

EPO FORM 1503 03.82 (P04C03)

Seite 2 von 2

**ANHANG ZUM EUROPÄISCHEN RECHERCHENBERICHT**
**ÜBER DIE EUROPÄISCHE PATENTANMELDUNG NR.**

EP 16 17 1275

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-07-2016

| Im Recherchenbericht angeführtes Patentdokument | Datum der Veröffentlichung | Mitglied(er) der Patentfamilie | | Datum der Veröffentlichung |
|---|---|---|---|---|
| KR 20030053484 A | 28-06-2003 | KR | 20020074443 A | 30-09-2002 |
| | | KR | 20030053484 A | 28-06-2003 |
| GB 2216522 A | 11-10-1989 | AT | 398073 B | 26-09-1994 |
| | | BE | 1003663 A3 | 19-05-1992 |
| | | CA | 1339147 C | 29-07-1997 |
| | | CH | 678851 A5 | 15-11-1991 |
| | | DE | 3907688 A1 | 21-09-1989 |
| | | ES | 2010439 A6 | 01-11-1989 |
| | | FI | 891144 A | 10-09-1989 |
| | | FR | 2628419 A1 | 15-09-1989 |
| | | GB | 2216522 A | 11-10-1989 |
| | | HU | 199775 B | 28-03-1990 |
| | | IT | 1229563 B | 04-09-1991 |
| | | JP | H024746 A | 09-01-1990 |
| | | JP | 2751068 B2 | 18-05-1998 |
| | | LU | 87470 A1 | 02-10-1990 |
| | | NL | 8900573 A | 02-10-1989 |
| WO 2006059169 A2 | 08-06-2006 | AT | 429236 T | 15-05-2009 |
| | | CA | 2588195 A1 | 08-06-2006 |
| | | CN | 101068540 A | 07-11-2007 |
| | | DK | 1830861 T3 | 27-07-2009 |
| | | EP | 1830861 A2 | 12-09-2007 |
| | | ES | 2326091 T3 | 30-09-2009 |
| | | HU | 227588 B1 | 28-09-2011 |
| | | JP | 4976307 B2 | 18-07-2012 |
| | | JP | 2008521877 A | 26-06-2008 |
| | | KR | 20070086689 A | 27-08-2007 |
| | | US | 2008131519 A1 | 05-06-2008 |
| | | WO | 2006059169 A2 | 08-06-2006 |
| GB 2216418 A | 11-10-1989 | BE | 1002890 A3 | 16-07-1991 |
| | | CA | 1334576 C | 28-02-1995 |
| | | CH | 678918 A5 | 29-11-1991 |
| | | DE | 3907649 A1 | 28-09-1989 |
| | | FI | 891145 A | 10-09-1989 |
| | | FR | 2628324 A1 | 15-09-1989 |
| | | GB | 2216418 A | 11-10-1989 |
| | | HU | 209973 B | 30-01-1995 |
| | | IT | 1229562 B | 04-09-1991 |
| | | JP | H01316316 A | 21-12-1989 |
| | | LU | 87471 A1 | 02-10-1990 |
| | | NL | 8900574 A | 02-10-1989 |
| WO 9516661 A1 | 22-06-1995 | AU | 1241295 A | 03-07-1995 |

EPO FORM P0461

Für nähere Einzelheiten zu diesem Anhang : siehe Amtsblatt des Europäischen Patentamts, Nr.12/82

Seite 1 von 2

**ANHANG ZUM EUROPÄISCHEN RECHERCHENBERICHT**
**ÜBER DIE EUROPÄISCHE PATENTANMELDUNG NR.**

EP 16 17 1275

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-07-2016

| Im Recherchenbericht angeführtes Patentdokument | Datum der Veröffentlichung | Mitglied(er) der Patentfamilie | Datum der Veröffentlichung |
|---|---|---|---|
| | | DE 69409969 D1 | 04-06-1998 |
| | | DE 69409969 T2 | 10-12-1998 |
| | | EP 0734373 A1 | 02-10-1996 |
| | | ES 2117390 T3 | 01-08-1998 |
| | | IT 1264987 B1 | 17-10-1996 |
| | | JP H09508619 A | 02-09-1997 |
| | | US 5750572 A | 12-05-1998 |
| | | WO 9516661 A1 | 22-06-1995 |
| JP H0977725 A | 25-03-1997 | KEINE | |

EPO FORM P0461

Für nähere Einzelheiten zu diesem Anhang : siehe Amtsblatt des Europäischen Patentamts, Nr.12/82

Seite 2 von 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0734373 B1
- EP 1260496 A1

- DE 3907649 C2

**In der Beschreibung aufgeführte Nicht-Patentliteratur**

- Official Methods and Recommended Practices of the AOCS. 2013